# EUROPEAN PATENT APPLICATION

(11) **EP 4 806 398 A1**
(43) Date of publication of application: **16.09.2026**
(21) Application number: 25162523.2
(22) Date of filing: 10.03.2025
(51) Int. Cl.: A61F 2/58, A61F 2/68, A61F 2/74

(54) **MOTION ASSISTANCE DEVICE, HAND MOTION ASSISTANCE DEVICE, AND MOTION ASSISTANCE SYSTEM**

(71) Applicant: Technische Universität München, in Vertretung des Freistaates Bayern, 80333 München (DE)
(72) Inventor: PIAZZA, Cristina, 85748 Garching (DE); GEISSENBERGER, Tobias, 81243 München (DE)
(74) Representative: Hoefer & Partner Patentanwälte mbB

(57) **Abstract**

The invention concerns a motion assistance device (1), comprising at least one fluid power actuator (2) configured to extend or contract based on input fluid pressure; at least one control valve (3) connected to the actuator(s) (2) and configured to input fluid pressure into the actuator(s) (2); wherein the at least one control valve (3) comprises a plurality of output ports (4), wherein each output port (4) is connected to one or more of the actuators (2), and wherein the at least one control valve (3) is configured to output fluid pressure sequentially via the output ports (4) based on an input pressure input into the at least one control valve (3). The invention also concerns a hand motion assistance device (100) and a motion assistance system (1000).

## Description

### Field of the Invention

The invention concerns a motion assistance device, a hand motion assistance device, and a motion assistance system.

### Background of the Invention

Motion assistance devices such as prosthetics for limbs of a user and exoskeletons in support of limbs for a user are known.

Commercially available prostheses can be separated into passive, body-actuated, and externally actuated prosthetics. Typically, for hand prosthetics, only externally actuated hand prosthetics offer several grasp modes and enhanced functionality. Challenges in different grasp modalities of hand prostheses or of different movement modalities for other limb prostheses arise from the complexity of decoding a user's intention. Capturing user's intentions to control prostheses with multiple modalities can require processing high-density EMG signals and exteroceptive sensor inputs. However, the necessity for such complex control methods limits the commercialization of prostheses with multiple modes, especially hand prostheses with multiple grasping modes, making them expensive, decreasing their robustness, and increasing cognitive burden on the user.

In general, the prior known motion assistance devices have several drawbacks, such as limited functionality, particularly in the exemplary case of hand motion assistance a limited grasp functionality (different combinations of fingers), and in the cases of higher functionality comprise a high number of components, which particularly need to be individually controlled. The necessity for individual control of many components, in addition to being expensive and commonly heavy, also strongly reduces a rate of acceptance and long-term use by the user due to high cognitive burden on the user.

### Summary

It is an object of the present invention to overcome these deficiencies. In particular, it is an object of the present invention to provide a motion assistance device, a hand motion assistance device, and a motion assistance system which have an advantageously increased degree of functionality, and which preferably are light-weight, cost-effective, and easy to use with a low cognitive burden.

The solution of these objects is achieved by the subject matter of the independent claim. The dependent claims contain advantageous embodiments of the present invention.

The solution of these objects is achieved by the motion assistance device according to claim 1. The motion assistance device comprises at least one fluid power actuator configured to extend or contract based on input fluid pressure. Further, the device comprises at least one control valve connected to the actuator(s) and configured to input fluid pressure into the actuator(s). Therein, the at least one control valve comprises a plurality of output ports. Each output port is connected to one or more of the actuators. Further, the at least one control valve is configured to output fluid pressure sequentially via the output ports based on an input pressure input into the at least one control valve.

Since the output of the fluid pressure sequentially via the output ports is based on an input pressure of the control valve, the control of the actuator(s) is strongly simplified, while simultaneously providing for a higher degree of functionality based on the multiple outputs of the control valve. Thus, the motion assistance device provides an advantageously increased degree of functionality while being easy to use, light-weight, and cost-effective.

In the present sense, "extend or contract based on input fluid pressure" preferably means that the respective actuator is configured to be able to only extend, or to be able to only contract, or be able to extend and (non-simultaneously) contract, based on input pressure. Preferably, the respective actuator is configured to contract with an increase in input pressure and afterwards extend with decrease in input pressure, or vice versa. For example, in hand-based applications, the respective actuator may contract with increase in input pressure for gripping, and extend with decrease in pressure for relaxing, while in arm-based or leg-based applications, extension with increased input pressure may preferably be employed.

Preferably, in the present sense, "plurality" refers to exactly two or more. Further preferably, "at least one" explicitly includes "exactly one" or a plurality.

Preferably, the term "connected" with reference to the actuator(s), the control valve(s), and in the following an input pressure generating device, refers to a fluid connection, wherein this connection may be "open" or "closed".

Preferably, the fluid power actuator is a pneumatic or a hydraulic actuator. In other words, the fluid is preferably air (pneumatic) or an incompressible liquid (hydraulic). For hand-based applications, the fluid power actuator is preferably a pneumatic actuator. Preferably, the pneumatic actuator is a soft pneumatic actuator, particularly preferably in hand-applications a soft pneumatic finger actuator. Such soft pneumatic finger actuators preferably contract with increased input pressure for gripping.

In the present sense, the term "output fluid pressure sequentially via the output ports" preferably refers to the control valve, which is preferably also referred to as a sequence valve, outputting to the output valves in a predetermined sequence, based on the structure and physical configuration of the control valve, as a function of the input pressure. The term "sequentially" in the present sense preferably refers to "successively", i.e. after one another. In this regard, it is to be understood that at least one (second) output port is sequentially after at least one other (first) output port, wherein for example another (third) output port is preferably sequentially after the first and second, or simultaneously with the first or the second output port. For example, a first output port, in sequence, can be opened, and then afterwards a second and a third output port are opened simultaneously, or all three can be opened successively such that none are opened simultaneously with one another. Thus, preferably, some or all output ports are successively opened, and in the former case (i.e. "some") preferably one or some other output ports may be opened simultaneously with one another. As above, the term "outputting to the output valves" will in the following also be referred to as "opening the output" or the like for the sake of brevity.

In the understanding herein, the term "sequentially" or the corresponding terms "sequence / in sequence" will be described with reference to rising input pressure. When referring to decreasing pressure these will be referred to as "opposite", for example "in opposite sequence / sequentially opposite", for easier understanding.

Furthermore, the term "based on an input pressure" preferably refers to thresholds in input pressure. For instance, in sequence of rising input pressure, a first output port is opened at a first threshold of input pressure (or normally open), and a second output port is opened at a second threshold of input pressure, and so on. As stated above, multiple ports may be opened at a single threshold (i.e. simultaneously with regard to sequence). Further, increases or decreases in input pressure within a range between two thresholds preferably do not open output ports. This has the advantage in that fluctuations in input pressure do not cause tremors in the actuators, for example. As will be explained below with regard to some examples, the thresholds are preferably predetermined via arrangement of the output ports, particularly a distance therebetween.

In the following, for the sake of brevity, "the control valve" will be referred to. It goes without saying that multiples or combinations of these are preferably included in the motion assistance device, or exactly only one of these is included therein. The same is preferably the case with respect to the actuator(s).

Preferably, the output of the control valve is configured to be based solely on the input pressure input into the control valve, especially apart from any constructive constants (for example, bias against input pressure, arrangement of output ports, etc.) thereof. Further preferably, no active control apart from inputting input pressure is performable on the control valve, especially no electrical control or otherwise further (second) input pressure. Thereby, the control valve and its control are simplified, reducing cognitive burden on the user and making the control valve more light-weight and cost-effective.

Preferably, the control valve sequentially opens a first output and then a second output, preferably following this opens further (third, fourth, etc.) outputs, wherein all foregoing opened outputs are kept open. For instance, when the third output is opened, also the first and second outputs remain open. Examples of such sequence valves are preferably cylinder-piston sequence valve type and poppet sequence valve type, which will be discussed below.

Preferably in addition or alternatively thereto, the control valve is configured to sequentially open one or more outputs while closing another. For instance, the control valve is preferably configured to sequentially open the first output, open the second output, and open the third output, while for instance closing the first or second output when opening the third output. Examples of such sequence valves are preferably spool type sequence valves, which will be discussed below.

Preferably, the output fluid pressure sequentially increases degree of actuation and/or force of actuation of the respective one or more actuators. In other words, with rising pressure, the degree of actuation is increased (extension or contraction) and/or the force of the actuation is increased. In the aforementioned hand application example, for instance, further open outputs can further contract the actuators and apply more force through the actuators. Preferably, if the respective actuator is (already) fully actuated with respect to degree of actuation (extension or contraction), further opened outputs connected thereto (only) further increase the force exerted thereby (i.e. "or": only more force). In an exemplary case of the actuator having an (internal) resistance or counter-force, especially linear with degree of actuation, for example via a spring in the actuator biased against input pressure, further opened outputs preferably increase degree of actuation, preferably without increasing force exerted thereby (i.e. "or": only more degree of actuation) as a balance between input pressure and counter-force.

Preferably, at least one of the control valve(s) is respectively connected to one actuator, and each output port of the respective control valve is connected to the respective actuator. Preferably, said control valve is connected to exactly one and only one actuator. Thereby, each sequentially opened output can increase degree of actuation and/or force of the actuation of that actuator individually, which advantageously increases degree of functionality with respect to one actuator.

Further preferably, at least one of the control valve(s) is respectively connected to a plurality of actuators. In combination with the above, one control valve is preferably connected to exactly one first actuator, while another second control valve is connected to at least a second actuator and a third actuator. Two control valves can also preferably be connected to the same actuator, for example the second control valve is preferably connected to at least a second actuator and the first actuator. Thereby, advantageously the degree of functionality with respect to multiple actuators is advantageously increased.

Preferably, in the control valve connected to a plurality of actuators, at least one output port of the control valve is connected to exactly one actuator.

Further preferably, in the control valve connected to a plurality of actuators, at least one output port is connected to a plurality of actuators. The two foregoing cases can be combined, such that one output port is connected to one actuator, and another output port is connected to multiple other (or the same) actuators. Alternatively, each output port is connected to exactly one actuator, while preferably at least one actuator is connected to multiple output ports (i.e. multiple output ports connected to one same actuator), or each actuator is connected to exactly one output port (i.e. same number of actuators and output ports). Preferably, connecting multiple output ports to one actuator comprises the possibility of the output ports being connected at different input pressure portions of that actuator. This has the advantage in further increasing the degree of functionality, as separate portions of each actuator can advantageously be individually controlled.

Preferably, the aforementioned connections are achieved via connection lines or connection tubes. The aforementioned connections are preferably respectively achieved directly via these lines. In other words, the lines are preferably directly connected between the control valve(s) and the actuator(s). Preferably, for an output port being connected to multiple actuators, a connection line thereof is branched, with one end being connected to the output port and an opposite side of the line comprising multiple ends branched from the aforementioned one end. Preferably, the motion assistance device does not comprise any further valves and/or manifolds in addition to the foregoing described control valve(s), particularly preferably not between foregoing described control valve(s) and actuator(s) or between foregoing described control valve(s) and an input pressure generating device supplying pressure to the control valve(s).

In some embodiments, at least one of the control valve(s) is a sequence valve comprising a cylinder and a piston, wherein the piston is biased against the input fluid pressure and with rising pressure sequentially connects the output ports to the input port. Preferably, this sequence valve corresponds to the aforementioned cylinder-piston sequence valve type. With this configuration, control of sequentially opening the output ports is advantageously simplified, as their sequential order is determined by their arrangement along a longitudinal extension of the cylinder. This has the advantage in being easy to use, lightweight and cost-effective while providing advantageously high degree of functionality.

Preferably therein, in rising input pressure sequence, a first output port is normally open and one or more further output ports, especially second and third output ports, are normally closed. In other words, with respect to the longitudinal extension, at zero or low input pressure, the piston is arranged between the first output port and the further output ports (second output port, etc.) such that the first output port is normally open. This has the advantage in that the one or more actuators connected to the first output port can be advantageously directly controlled via the input pressure, since this input pressure is directly output to said one or more actuators of the first output port. Thereby, movement of these actuators is essentially instantaneously started, and there is no time lag between a user's intention (input pressure) and start of movement, allowing for high degree of functionality and ease of use as well as low cognitive burden.

Preferably, the cylinder-piston type sequence valve further comprises a spring. Therein, the cylinder and piston variably define an input chamber into which the input pressure is input. In other words, via movement of the piston in reaction to input pressure, the input chamber has a variable volume directly correlated with input pressure. The spring is disposed between the cylinder and piston on a side of the piston opposite the input chamber. In other words, the spring provides the aforementioned bias against input pressure.

In some embodiments, the spring may be replaced by or supplemented with one or more other biasing elements such as another spring , (compressed) air or liquid, (electro-)magnetics, or the like. Thereby, bias against input pressure can also advantageously be predetermined as non-linear or with steps, advantageously increasing degree of functionality.

Preferably, the control valve comprises exactly and only one input chamber. Although multiple input pressure lines may be connected to the input chamber, preferably only exactly one input line is connected to the input chamber of the control valve. Thereby, the entire control of the control valve is preferably based solely on one input pressure. This advantageously simplifies the control of the control valve as well as its constructions, and provides for the device to be light-weight and cost-effect as well as cause a low burden on a user.

Preferably, in some embodiments, a spring constant and size of the spring are predetermined and preferably enclosed entirely within the cylinder. Thereby, the spring is adapted to the corresponding application and not easily modifiable by the user. This has the advantage in that an amount of input and modification possibilities for the user is decreased, further advantageously reducing cognitive burden on the user.

On the other hand, preferably in some embodiments, the cylinder comprises a through-hole with a turnable adjustment element, such as a screw, connected to the spring and configured to compress or decompress the spring when turned. This allows for individual adjustment or fine-tuning by the user especially to different uses or applications, for example reducing the input pressure necessary for overcoming the bias, which also reduces the input pressure output to the output ports and thus reduces the force of actuation and/or degree of actuation.

Preferably, the sequence valve further comprises an ambient air port, i.e. an input port for ambient air pressure. The ambient air port may also be referred to as a ventilation port. Therein, the cylinder and piston variably define an ambient chamber connected to the ambient air port. The piston seals the ambient chamber from the input chamber. For instance, the piston comprises a dynamic sealing ring, which separates the control valve into the two chambers. The ambient chamber preferably equally contracts as the input chamber expands and vice versa. Thereby, preferably, one or more output ports are opposite sequentially connected to ambient air, i.e. when not connected to the input chamber. Thereby, the actuators (currently) not being actuated are advantageously relaxed, lowering effort and cognitive burden on the user.

Advantageously, the piston of the sequence valve seals the output ports from the ambient chamber in the sequence, i.e. the same input pressure rising sequence, of connecting the output ports to the input port. In other words, preferably, when an output port is opened, it is sealed from the ambient chamber, and vice versa. Thereby, especially in opposite sequence, the actuators are advantageously sequentially relaxed, which further increases degree of functionality, while also lowering effort and cognitive burden on the user.

In some preferable embodiments, the device comprises five actuators and the control valve comprises three output ports. Therein, in rising input pressure sequence, of the control valve, a first output port is connected to a first actuator and a second actuator, a second output port is connected to a third actuator, and a third output port is connected to a fourth and a fifth actuator. Preferably, the foregoing connections are exclusive, i.e. the first output port is connected only to the first actuator and the second actuator, etc. This has the advantage in that actuators can be grouped with regard to different input pressures, i.e. multiple actuators being activated at certain input pressures, which increases degree of functionality while also simplifying control.

In further preferable embodiments, the control valve is a spool-type sequence valve. Therein, similar to the piston, the sequence valve comprises a spool within the cylinder. The spool comprises multiple sealing portions comparable to multiple pistons. Thereby, for example, when one output port is opened, one or more other port(s) may be closed. In particular, the one or more other port(s) are blocked by the spool, and not connected to ambient air thereby. This has the particular advantage in that some output ports and corresponding actuator(s) can be closed and thereby kept at their corresponding (prior) input pressure, while other(s) are opened at and with higher input pressures. This advantageously increases degree of functionality. For example, in an illustrative example of a hand motion assistance device, an actuator corresponding with/to a thumb finger may be actuated at a first input pressure threshold and then blocked, so as to prevent said actuator exerting a high force at higher pressures, at which actuation of for example middle finger or ring finger would be suitable.

The present invention also concerns a hand motion assistance device. The hand motion assistance device is preferably a hand prosthetic or a hand exoskeleton. The hand motion assistance device comprises the motion assistance device of any one or a combination of the foregoing described examples and explanations. As a hand prosthetic, the fluid power actuators are integrated in prosthetic finger members thereof. As a hand exoskeleton, the fluid power actuators are configured to be externally attachable to fingers of a user, for example via a wearable glove.

Preferably, the hand motion assistance device comprises the motion assistance device of the foregoing comprising five actuators, with the control valve comprising three output ports. In this example, the first actuator corresponds to a thumb, the second actuator corresponds to an index finger, the third actuator corresponds to a middle finger, the fourth actuator corresponds to a ring finger, and the fifth actuator corresponds to a little finger of the hand prosthetic or of the user's hand. The term "corresponds to" means that in a hand prosthetic, it acts as said finger, and in an exoskeleton it supports said finger. Thereby, different advantageous grip modalities are defined.

With the output ports in corresponding input pressure sequence, a first grip mode and input pressure actuates the thumb and index finger (first output port open), a second grip mode actuates thumb, index, and middle finger (first and second output ports open), and a third grip mode actuates the foregoing as well as the ring finger and the little finger (all three output ports open) with rising pressure. This has been demonstrated as advantageous for degree of functionality as well as ease of use and lower burden, cost-effectiveness and weight of the device.

The present invention also concerns a motion assistance system. The motion assistance system comprises a motion assistance device according to any of the foregoing examples or combinations thereof, or a hand motion assistance device according to the above. The system further comprises a control device that is connected, especially electrically, to one or more bioelectric and/or myoelectric sensors for sensing signals from muscles and/or nerves of a user. The control device is further configured to calculate a target input pressure to be input into the control valve(s) based on the sensed signal. Further, the system comprises an input pressure generating device, especially a compressor or a pump, configured to supply fluid pressure to the control valve(s) based on the target input pressure. Thereby, a simple, light-weight and cost-effective motion assistance system is achieved with a high degree of functionality and for a wide range of applications.

Preferably, the input pressure generating device is integrated with the control valve in a common housing, or is provided separately therefrom. For example, in hand prosthetics/exoskeleton applications, it is preferable to dispose the input pressure generating device separately, and include a pressure line connecting the input pressure generating device with the input chamber of the control valve, the control valve preferably being located on the hand or wrist region of the prosthetic or of the user.

Although in the foregoing predominantly hand prosthetics/exoskeletons have been explained, it is to be understood that the devices and systems discussed in the foregoing are preferably suitable for a range of applications in prosthetics/exoskeletons for support of different limbs or extremities.

The control device preferably comprises a CPU, GPU, FPGA, SoC, or the like and additionally preferably comprises a storage medium, on which the foregoing described functions of the control device are preferably stored as computer implemented steps.

The foregoing described preferable embodiments and configurations may be combined.

Further details, advantages, and features of the preferred embodiments of the present invention are described in detail with reference to the figures. Therein:

### Brief Description of the Drawings

Fig. 1 shows a schematic overview of a motion assistance system comprising a motion assistance device according to an embodiment of the present invention;
Fig. 2 shows a detailed view of the motion assistance system of Fig. 1;
Figs. 3a,b show a detailed view explaining a first mode of operation of the motion assistance system of Fig. 1;
Figs. 4a,b show a detailed view explaining a second mode of operation of the motion assistance system of Fig. 1; and
Figs. 5a,b show a detailed view explaining a third mode of operation of the motion assistance system of Fig. 1.

### Description of the Embodiment

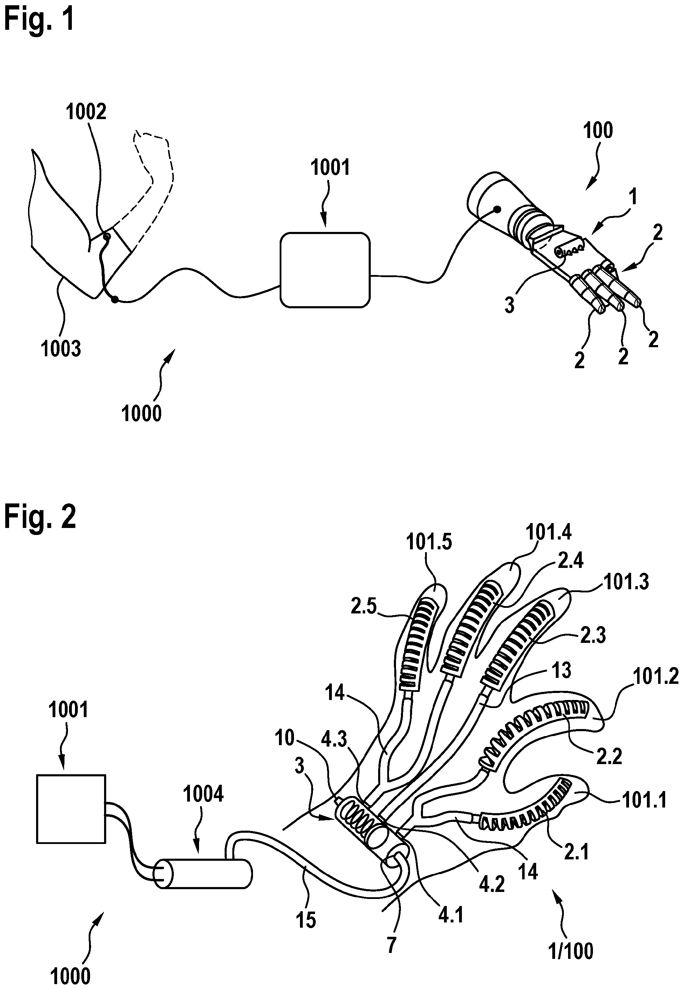

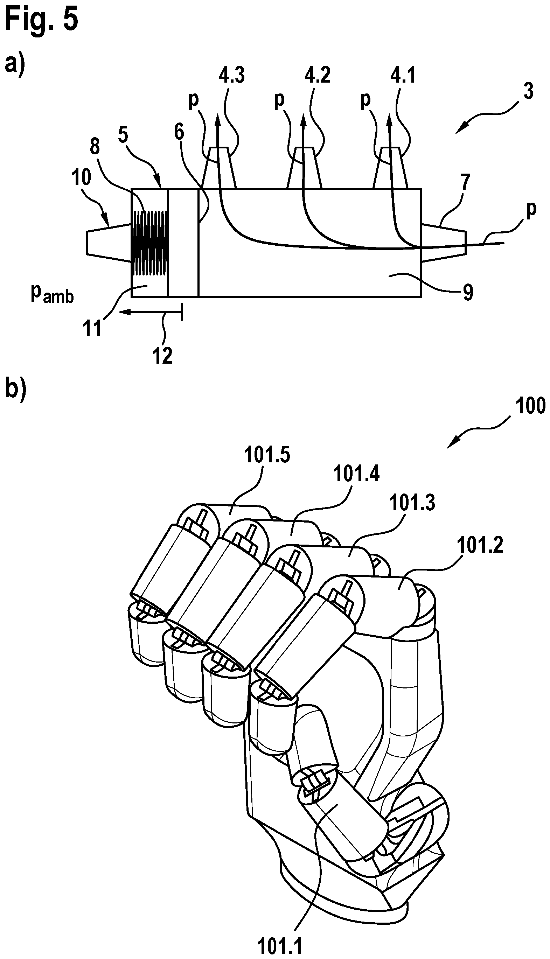

An embodiment of the present invention will be described with reference to Fig. 1 to 5a,b.

In the following, an embodiment of the present invention is explained for an application as hand prosthetic. The same principles are generally also to be understood for applications as/in a hand exoskeleton, and for applications for other limbs and extremities, likewise as prosthetics and/or exoskeletons.

First, an overview of the embodiment of the present invention will be explained with reference to Figs. 1 and 2, wherein Fig. 1 shows a schematic overview of a motion assistance system 1000 comprising a motion assistance device 1 according to an embodiment of the present invention, and Fig. 2 shows a detailed view of the motion assistance system 1000 of Fig. 1.

As can be taken from Fig. 1, the motion assistance system 1000 comprises a hand motion assistance device 100, which comprises a motion assistance device 1. The hand motion assistance device 100 of the present embodiment is a hand prosthetic. It is to be understood that the hand motion assistance device 100 does not necessarily need to be a full hand prosthetic, but can also replace only one or more fingers of a user.

Furthermore, the motion assistance system 1000 comprises a control device 1001. The control device 1001 is connected to a myoelectric sensor 1002 which in the present example is on one or more muscles of a user 1003. The control device 1001 measures myoelectric or muscle signals from the user 1003 which signals their intent to for instance close their fist. The control device 1001 calculates from this sensed signal a target input pressure (p) for inputting pressure into the hand motion assistance device 100, as will be explained in more detail with respect to Fig. 2.

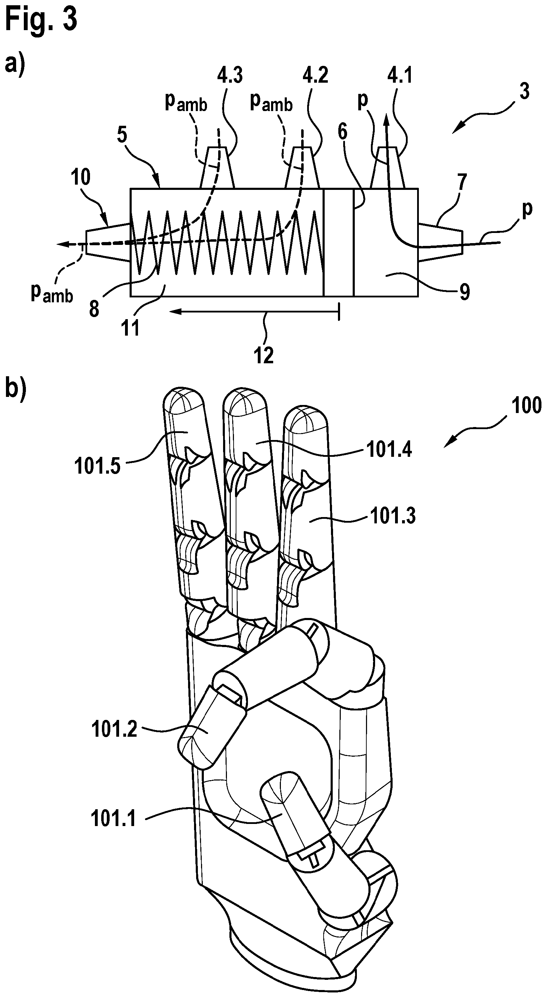

As can be seen in more detail in Fig. 2, the hand motion assistance device 100 comprises a motion assistance device 1. The motion assistance device 1 generally comprises one or more, in the present embodiment exactly five, fluid power actuator(s) 2, 2.1 - 2.5, which are configured to extend or contract based on input fluid pressure p (see also Fig. 3a).

Further, the motion assistance device 1 comprises one or more, in the present embodiment exactly one, control valve 3 connected to the actuator 2.1 - 2.5 and configured to input fluid pressure p into the actuator 2.1 - 2.5.

Therein, the control valve 3 comprises a plurality of output ports 4. Each output port 4 is connected to one or more of the actuators 2.1 - 2.5. Further, the control valve 3 is configured to output fluid pressure p sequentially via the output ports 4 based on an input pressure p input into the control valve 3. These aspects will be explained in greater detail with regard to Figs. 3a - 5b.

With further view on Fig. 2, the hand motion assistance device 100 is a hand prosthetic, and at least the actuators 2 of the motion assistance device 1 are integrated in finger members 101 of the hand motion assistance device 100. Preferably, in this example, the actuators 2 are soft finger actuators. Each finger member 101 of the present embodiment comprises one actuator 2.

In the present embodiment, the actuators 2 are driven by air pressure, i.e. are pneumatic actuators 2. The motion assistance system 1000 comprises an input pressure generating device 1004, which in the present embodiment is a compressor 1004. The compressor 1004 is connected to the control valve 3 and drives/controls actuation of the actuators 2 via input pressure calculated by the control device 1001 electrically connected to the compressor 1004. Herein, the compressor 1004 is directly connected to the control valve 3 via a single input line 15, with no further valves or manifolds, etc. therebetween.

In a modified embodiment, the actuators 2 may be driven by a pressurized liquid, i.e. hydraulic, in which case the input pressure generating device 1004 may be a pump.

In the present embodiment, the motion assistance device 1 comprises five actuators 2/2.1 - 2.5 which are each integrated in a finger member 101/101.1 - 101.5.

The control valve 3, as a comparison of Fig. 2 and Fig. 3a demonstrates, comprises herein three output ports 4.1, 4.2, 4.3. A first output port 4.1 is connected in common to the first actuator 2.1 and to the second actuator 2.2, which herein correspond to a thumb 101.1 and an index finger 101.2. A second output port 4.2 is connected only to the third actuator 2.3, which herein corresponds to a middle finger 101.3. A third output port 4.3 is connected in common to the fourth actuator 2.4 and to the fifth actuator 2.5, which herein correspond to a ring finger 101.4 and to a little finger 101.5 (pinky finger).

Further, as shown in Fig. 2, the aforementioned connections between the output ports 4.1, 4.2, 4.3 and the corresponding actuators 2.2 - 2.5 are achieved via connection lines or tubes 13, 14. Particularly, these connections are formed, apart from the connection lines 13, 14, directly between the output ports 4.1, 4.2, 4.3 and the actuators 2.2 - 2.5, with preferably no other or further elements therebetween such as further valves or manifolds, etc. In other words, the connection lines 13, 14 are connected directly between the output ports 4.1, 4.2, 4.3 and the corresponding actuators 2.2 - 2.5.

The aforementioned multiple connections of the first output port 4.1 to the first and second actuator 2.1, 2.2 and of the third output port 4.3 to the fourth and fifth actuator 2.4, 2.5 are achieved respectively via branched lines 14. The aforementioned single connection of the second output port 4.2 to only the third actuator 2.3 is achieved via a direct line 13.

With this grouping, as will be further evident from Figs. 3a - 5b, the present example of the motion assistance device 1 achieves a high degree of functionality, particularly three grip modes, and is easy for a user to use. Further, the present device 1 is lightweight and cost-effective.

The output ports 4.1 - 4.3 are driven sequentially based on the input pressure p. This will now be explained with reference to Figs. 3a - 5b. Therein, Figs. 3a, 4a, and 5a each show a detailed view of the control valve 3 in respectively different modes, whereas Figs. 3b, 4b, and 5b each show a corresponding view of the finger members 101.

The control valve 3 is a sequence valve and sequentially, depending on the input pressure p, opens outputs 4.1, 4.2, 4.3, i.e. connects them to the corresponding actuator(s) 2. For the sake of easier understanding, in the following, the term "sequentially" or "in sequence" will refer to rising input pressure p, as also shown in Fig. 3a with arrow 12 indicating positive sequence direction along a substantial longitudinal extension direction of the control valve 3. Description of "sequentially opposite" or "in opposite sequence" or the like will refer to a corresponding sequence with falling input pressure p, i.e. opposite the arrow 12.

In the present embodiment, the output ports 4.1 - 4.3 are arranged such that the control valve 3 opens these consecutively, i.e. one after the other. However, although not shown, the arrangement of the output ports 4.1 - 4.3 is not strictly limited to such a sequence.

The control valve 3 comprises a cylinder 5 and a piston 6. The control valve 3, specifically the cylinder 5, comprises an input port 7 for inputting input pressure p, an ambient air port 10 for inputting ambient air. A volume between the piston 6 and the input port 7 will be referred to as an input chamber 9. A volume between the piston 6 and the ambient air port 10 will be referred to as an ambient air chamber 11. The output ports 4.1 - 4.3 are also formed in the cylinder 5, i.e. in the same cylinder 5 as the aforementioned input port 7 and ambient air port 10.

The control valve 3 further comprises a biasing element for biasing the piston 6 against the input pressure p. In the present embodiment, the biasing element is a spring 8.

The piston 6 seals the input chamber 9 from the ambient air chamber 11, i.e. prevents fluid connection thereof. When air of input pressure p is input into the input port 7, the input chamber 9 is pressurized. In dependence on constructive constants of the control valve 3, such as surface area of the piston 6, spring constant and normal extension (natural extension) of the spring 8, and pressure of the ambient air chamber 11, the input pressure p presses the piston 6 in sequence direction 12 and thereby compresses the spring 8.

A further constructive constant of the control valve 3, namely an individual arrangement of the output ports 4.1 - 4.3 with regard to the cylinder 5 in combination with the above constants predetermine at which absolute input pressure p they open. Further, their relative arrangement to one another, such as their distance in sequence direction 12, determine relative input pressure differences between opening one output port 4.1 and another output port 4.2.

When input pressure p exceeds a predetermined threshold, the piston 6 is moved past an output port 4.1 - 4.3, and thereby the corresponding output port 4.1 - 4.3 is connected to the input chamber 9 and thus supplied with the input pressure p.

In the present example, the first output port 4.1 is arranged with respect to the piston 6, or vice versa, such that the first output port 4.1 is normally open. This has the advantage in that the first actuator 2.1 and the second actuator 2.2 are directly controlled via the input pressure p, which can decrease lag time between intention and actuation, thus decreasing cognitive effort.

On the other hand, however, the first output port 4.1 may also be arranged such that when the piston 6 is in its resting position, the first output port 4.1 is closed, i.e. separated from the input chamber 9. Thereby, tremors of the corresponding actuators 2.1, 2.2 in rest, for example if the input chamber 9 is (slightly) pressurized, can be advantageously prevented.

In the following, "p" denoted input pressure, "pamb" denoted ambient air pressure, "A" denotes surface area of the piston 6, "k" denotes the spring constant of the spring 8, "x0" denotes resting position of the piston 6, "x1" denotes a position of the first output port 4.1, "x2" denotes the position of the second output port 4.2, and "x3" denotes the position of the third output port 4.3.

If (p - pamb)A < k(x1 - x0):
In the normally closed example, the piston 6 separates the input port 7 and the output ports 4.1 - 4.3. The output ports 4.1 - 4.3 are all connected to the ambient air port 10, which supplies the actuators 2 with ambient pressure pamb. Consequently, the hand prosthesis 100 remains in an unactuated open position.

In the normally open example, the first output port 4.1 is open and is connected to the input pressure p.

In Figs. 3a and 3b, a first grip mode is demonstrated.

Herein, a relatively low input pressure p is input into the control valve 3, and the first actuator 2.1 and the second actuator 2.2, which are connected to the first output port 4.1 (see also Fig. 2), are actuated. This corresponds to: (p - pamb)A > k(x1 - x0).

Thereby, the thumb 101.1 and the index finger 101.2 are closed and exert the input pressure p.

The second output port 4.2 and the third output port 4.3 are meanwhile connected to the ambient air chamber 11 and are thus relaxed.

This grip mode, also referred to as a "pinch grasp" for precision grasping, is for example driven with equal to or less than 200 kPa.

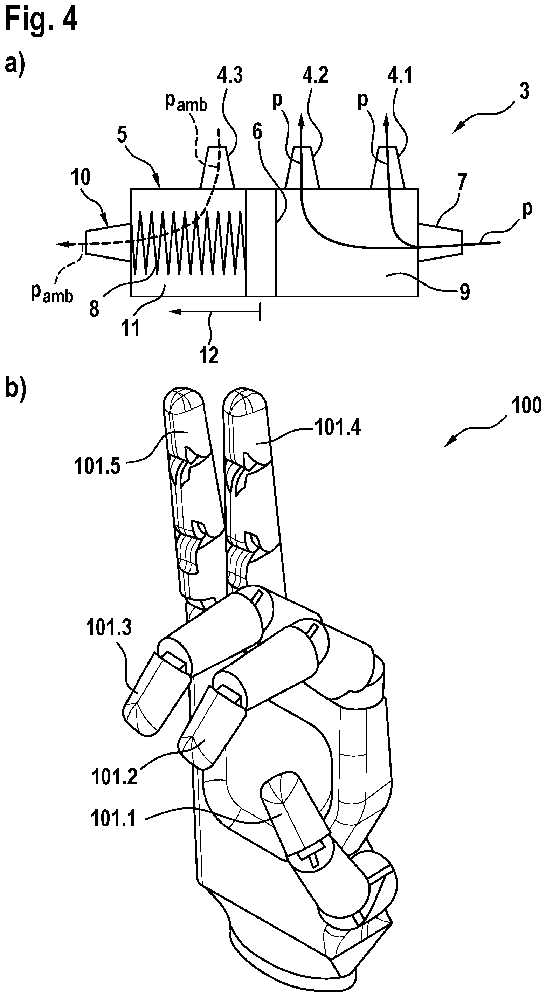

In Figs. 4a, b, a second grip mode, also referred to as "tripod grasp" is shown. As can be seen in Fig. 4a, the input pressure p is increased with regard to Fig. 3a,b, and the piston 6 is moved, in sequence direction 12, past the second output port 4.2. This corresponds to: (p-pamb)A > k(x2-x0).

Thereby, the third actuator 2.3 is actuated, and thus middle finger member 101.3 is also closed. The third output port 4.3 is further connected to the ambient air chamber 11.

This control further has the advantage in that between different thresholds of the output ports 4.1 - 4.3, fluctuations of input pressure p do not cause a tremor in the actuators 2, which greatly enhances comfort and ease of use, and reduces cognitive effort.

In this example, this second grip mode is driven with 200 - 300 kPa.

In Figs. 5a, 5b, a third grip mode, also referred to as "power grasp" is shown. As can be seen in Fig. 5a, the input pressure p is further increased with regard to Fig. 4a,b, and the piston 6 is moved, in sequence direction 12, past the third output port 4.3. This corresponds to: (p - pamb)A > k(x3 - x0).

Thereby, the fourth actuator 2.4 and the fifth actuator 2.5 connected to the third output port 4.3 are actuated, and thus the ring finger 101.4 and the little finger 101.5 are also closed. None of the output ports 4.1 - 4.3 are connected to the ambient air chamber 11, instead all being connected to the input chamber 9.

In this example, this third grip mode is driven with over 300 kPa. Of course, the foregoing pressure examples are merely exemplary for explaining different thresholds and functions of the invention, are rough estimates, and can be adapted, especially via the foregoing explained constructive constants, to different applications or users.

The applied air pressure p at the input port 7 balances the force generated by the spring 8 and ambient air pressure pamb at the ambient air port 10. Since the spring force depends on its compression, the spring 8 is compressed when applying high pressure at the inlet. Consequently, the length of the spring 8 correlates with the applied air pressure p. By choosing the locations of the outlet ports 4, in the present example on a top of the cylinder 5 accordingly, the required input pressure p to connect the outlet ports 4 to the pressure supply (compressor 1004) can be customized.

As demonstrated in Figs. 3a - 5b, by choosing spring stiffness and geometrical control valve 3 dimensions, the input pressure points at which the control valve 3 switches from configuration of Fig. 3a to Fig. 4a to Fig. 5a are customized. Reducing the input pressure vice-versa moves the piston 6 in the opposite sequence direction and connects the output ports 4 subsequently to the ambient air port 10. Thus, a change from power grasp to tripod grasp and finally to pinch posture can be achieved. Furthermore, choosing a spring 8 with a non-constant stiffness k(x-x0) introduces the possibility of different transitions between grasp modality activation points. Thus, the maximum forces during precision grasping, tripod grasping and power grasping can be modified according to the required application.

In the exemplary application of the present embodiment, of a pneumatic hand prosthesis 100, the first output port 4.1 is connected to the thumb and index actuators 2.1, 2.2, output port 4.2 to the middle finger actuator 2.3 and output port 4.33 to the actuators 2.4, 2.5 of the ring finger and little finger 101.4, 101.5.

By choosing this connection pattern, switch configuration of Fig. 3a activates the thumb and index to form a pinch grasp; switch configuration of Fig. 4a activates thumb, index and middle finger to form a tripod grasp; and switch configuration of Fig. 5a activates all fingers to form a power grasp.

The control valve 3 of the present embodiment is only controlled with a single input, namely the input pressure p. All switch patterns (modes of operation) are activated based on the intensity of the inlet pressure p. Such a simple control mechanism is easy to control, for instance, based on the intensity of myoelectric signals. Using a single myoelectric muscle activation signal preferably suffices to control the inlet pressure p and implicitly switch grasp modes.

A potential user could show small muscle activation to command a low pressure and activate a pinch grasp, a medium muscle activation for a tripod grasp or a high activation for a power grasp. By design of the control valve 3, muscle activation preferably correlates with the inlet pressure p and grasp force since a high inlet pressure p will preferably lead to high grasp forces based on the working principle of the pressure driven hand prosthesis.

Thus, the user can intuitively grasp with a low-force pinch grasp in case of small muscle activation, a medium-force grasp tripod grasp for medium muscle activation or a high-force power grasp for high muscle activation. This type of grasping behavior is intuitive for human grasp strategies since delicate objects, e.g. raspberries, are usually grasped with a pinch grasp, while heavy objects, e.g. bottles, are grasped with a power grasp.

The foregoing described embodiment is also to be understood as applicable to other limbs and/or extremities. For example, the actuators 2 can be implemented in/on arms or legs, with each actuator 2 for example controlling further bending or stronger bending thereof, or for example being connected to an arm and a wrist, allowing for successive bending of an elbow and a wrist, or knee and ankle, etc.

Thereby, the present embodiment provides a motion assistance device 1, a hand motion assistance device 100, and a motion assistance system 1000 which respectively have an advantageously increased degree of functionality, and which are light-weight, cost-effective, and easy to use with a low cognitive burden for a user.

In addition to the foregoing written explanations, it is explicitly referred to figures 1 to 5b, wherein the figures in detail show configuration examples of the invention.

### List of Reference Numerals

- 1: motion assistance device
- 2: fluid power actuator
- 3: control valve
- 4: output port
- 5: cylinder
- 6: piston
- 7: input port
- 8: spring
- 9: input chamber
- 10: ambient air port
- 11: ambient chamber
- 12: sequence direction with rising input pressure
- 13: direct line
- 14: branched line
- 15: input line
- 100: Hand motion assistance device
- 101: prosthetic finger member
- 1000: Motion assistance system
- 1001: control device
- 1002: myoelectric sensor
- 1003: user
- 1004: input pressure generating device

## Claims

1. Motion assistance device (1), comprising:
at least one fluid power actuator (2) configured to extend or contract based on input fluid pressure;
at least one control valve (3) connected to the actuator(s) (2) and configured to input fluid pressure into the actuator(s) (2); wherein
the at least one control valve (3) comprises a plurality of output ports (4), wherein each output port (4) is connected to one or more of the actuators (2), and wherein the at least one control valve (3) is configured to output fluid pressure sequentially via the output ports (4) based on an input pressure input into the at least one control valve (3).

2. Motion assistance device (1) according to claim 1, wherein the sequentially output fluid pressure sequentially increases degree of actuation and/or force of actuation of the respective one or more actuators (2).

3. Motion assistance device (1) according to any one of the foregoing claims, wherein at least one of the control valve(s) (3) is respectively connected to one actuator (2), and each output port (4) of the respective control valve (3) is connected to the respective actuator (2).

4. Motion assistance device (1) according to any one of the foregoing claims, wherein at least one of the control valve(s) (3) is respectively connected to a plurality of actuators (2).

5. Motion assistance device (1) according to claim 4, wherein at least one output port (4) of the respective control valve (3) is connected to exactly one actuator (2).

6. Motion assistance device (1) according to claim 4 or claim 5, wherein at least one output port (4) of the respective control valve (3) is connected to a plurality of actuators (2).

7. Motion assistance device (1) according to any one of the foregoing claims, wherein at least one of the control valve(s) (3) is a sequence valve comprising a cylinder (5) and a piston (6), wherein the piston (5) is biased against the input fluid pressure and with rising pressure sequentially connects the output ports (4) to an input port (7) for the input pressure.

8. Motion assistance device (1) according to claim 7, wherein, in rising input pressure sequence, a first output port (4.1) is normally open and one or more further output ports (4.2, 4.3), especially second and third output ports (4.2, 4.3), are normally closed.

9. Motion assistance device (1) according to claim 7 or claim 8, wherein the sequence valve (3) further comprises a spring (8), wherein the cylinder (5) and piston (6) variably define an input chamber (9) into which the input pressure is input, and wherein the spring (8) is disposed between the cylinder (5) and piston (6) on a side of the piston (6) opposite to the input chamber (9).

10. Motion assistance device (1) according to claims 7 to 9, wherein the sequence valve (3) further comprises an ambient air port (10), wherein the cylinder (5) and piston (6) variably define an ambient chamber (11) connected to the ambient air port (10).

11. Motion assistance device (1) according to claim 10, wherein the piston (6) seals the output ports (4) from the ambient chamber (11) in the sequence of connecting the output ports (4) to the input port (7).

12. Motion assistance device (1) according to any one of the foregoing claims, comprising five actuators (2.1, 2.2, 2.3, 2.4, 2.5), wherein the control valve (3) comprises three output ports (4.1, 4.2, 4.3), wherein, in rising input pressure sequence, of the control valve (3), a first output port (4.1) is connected to a first actuator (2.1) and a second actuator (2.2), a second output port (4.2) is connected to a third actuator (2.3), and a third output port (4.3) is connected to a fourth (2.4) and a fifth actuator (2.5).

13. Hand motion assistance device (100), especially a hand prosthetic or a hand exoskeleton, comprising the motion assistance device (1) according to any one of the foregoing claims, wherein the fluid power actuators (2) are integrated in prosthetic finger members (101) of the hand prosthetic or are configured to be externally attachable to fingers of a user as an exoskeleton.

14. Hand motion assistance device (100) according to claim 13 comprising the motion assistance device (1) according to claim 12, wherein the first actuator (2.1) corresponds to a thumb (101.1), the second actuator (2.2) corresponds to an index finger (101.2), the third actuator (2.3) corresponds to a middle finger (101.3), the fourth actuator (2.4) corresponds to a ring finger (101.4), and the fifth actuator (2.5) corresponds to a little finger (101.5) of the hand prosthetic or of the user's hand.

15. Motion assistance system (1000) comprising a motion assistance device (1) or a hand motion assistance device (100) according to any one of the foregoing claims, further comprising:
a control device (1001) connected, especially electrically, to one or more bioelectric and/or myoelectric sensors (1002) for sensing signals from muscles and/or nerves of a user (1003), and configured to calculate a target input pressure to be input into the control valve(s) (3) based on the sensed signal; and
an input pressure generating device, especially a compressor (1004) or a pump, configured to supply fluid pressure to the control valve(s) (3) based on the target input pressure.
